(19) **Europäisches Patentamt / European Patent Office / Office européen des brevets**

(11) **EP 4 730 352 A1**

(12) **EUROPEAN PATENT APPLICATION**

(43) Date of publication:
**22.04.2026 Bulletin 2026/17**

(21) Application number: **25208227.6**

(22) Date of filing: **13.10.2025**

(51) International Patent Classification (IPC):
**G16H 30/40** (2018.01)   **A61B 6/50** (2024.01)
**G06F 18/214** (2023.01)   **G06N 3/08** (2023.01)
**G06T 7/00** (2017.01)   **G06V 10/70** (2022.01)
**G06V 40/14** (2022.01)   **G16H 50/20** (2018.01)
**G16H 50/50** (2018.01)   **G16H 50/70** (2018.01)

(52) Cooperative Patent Classification (CPC):
**G16H 30/40; A61B 6/504; G06F 18/214;
G06N 3/045; G06N 3/08; G06T 7/0012; G06T 7/11;
G06V 10/70; G06V 40/14; G16H 50/20;
G16H 50/50; G16H 50/70;** G06T 2207/10072;
G06T 2207/20081; G06T 2207/20084;     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **16.10.2024 US 202418916992**

(71) Applicant: **Siemens Healthineers AG
91301 Forchheim (DE)**

(72) Inventors:
• **XIE, Long
Princeton, NJ, 08540-6632 (US)**
• **GIBSON, Eli
Princeton, NJ, 08540-6632 (US)**
• **GEORGESCU, Bogdan
Princeton, NJ, 08540-6632 (US)**

(74) Representative: **HKW Intellectual Property PartG
mbB
Theresienhöhe 12
80339 München (DE)**

(54) **DEEP REINFORCEMENT LEARNING BASED ROBUST NEUROVASCULAR MAPPING**

(57)     Systems and methods for generating a patient-specific vascular tree are provided. 1) one or more medical images of a patient and 2) a vascular tree template comprising a plurality of points are received. The plurality of points of the vascular tree template are iteratively adjusted based on the one or more medical images using one or more AI (artificial intelligence) agents to generate a patient-specific vascular tree for the patient. The patient-specific vascular tree is output.

**FIG. 1**

100

Receive 1) one or more medical images of a patient and 2) a vascular tree template comprising a plurality of points
102

Iteratively adjust the plurality of points of the vascular tree template based on the one or more medical images using one or more AI agents to generate a patient-specific vascular tree for the patient
104

Output the patient-specific vascular tree
106

EP 4 730 352 A1

(52) Cooperative Patent Classification (CPC): (Cont.)
G06T 2207/30016; G06T 2207/30101

**Description**

TECHNICAL FIELD

The present invention relates generally to AI/ML (artificial intelligence/machine learning) based medical imaging analysis, and in particular to deep reinforcement learning based robust neurovascular mapping.

BACKGROUND

Neurovascular trees of the brain of patients are extracted from medical images to provide for a detailed mapping of the blood vessels of the brain. Such neurovascular trees are important for the visualization and localization of neurovascular abnormalities, as well as for devising plans for medical interventions. However, the extraction of neurovascular trees is challenging due to the high complexity of the human neurovascular system. A typical neurovascular tree of the anterior and posterior neurovascular systems encompasses more than 15 segments, which exhibit significant anatomical variability across different individuals. Further, vessels can be easily obscured by bones in medical imaging, complicating their identification. The presence of abnormalities, such as large vessel occlusion and aneurysms, further increases the complexity.

Recently, AI/ML based approaches have been proposed for neurovascular tree mapping. However, there is still considerable room for improvement in such conventional AI/ML based neurovascular tree mapping approaches in terms of accuracy, robustness, and preservation of neurovascular topology.

BRIEF SUMMARY OF THE INVENTION

The invention is defined by the attached set of claims. Further details of the disclosed method, devices and system are described below, which are helpful for understanding the claimed invention.

In accordance with one or more embodiments, systems and methods for generating a patient-specific vascular tree are provided. 1) one or more medical images of a patient and 2) a vascular tree template comprising a plurality of points are received. The plurality of points of the vascular tree template are iteratively adjusted based on the one or more medical images using one or more AI (artificial intelligence) agents to generate a patient-specific vascular tree for the patient. The patient-specific vascular tree is output.

In one embodiment, the plurality of points is jointly adjusted at each iteration. The plurality of points may be adjusted in at least one of a left direction, a right direction, an inferior direction, a superior direction, a posterior direction, or an anterior direction. The one or more AI agents receives as input at least one of the one or more medical images corresponding to a current position of the one or more AI agents and generates as output a vector corresponding to the adjustments for the plurality of points.

In one embodiment, the one or more medical images comprise at least one of one or more bone removed medical images or one or more vessel probability maps.

In one embodiment, the one or more AI agents are trained using synthesized large vessel occlusion data. The synthesized large vessel occlusion data is generated by simulating an occlusion in a particular segment of a vessel tree by removing the particular segment from the vessel tree and, in response to determining that the particular segment is a sole bloody supply to a downstream segment of the vascular tree, removing the downstream segment from the vascular tree.

In one embodiment, the vascular tree template is generated by averaging a set of manually identified vascular trees for a patient population. A vascular tree is selected from the set of manually identified vascular trees. The remaining vascular trees of the set of manually identified vascular trees is aligned to the selected vascular tree. Positions of each vascular landmark of the aligned vascular trees are averaged. The set of manually identified vascular trees is aligned to the average position of each vascular landmark.

In one embodiment, the one or more medical images are computed tomography angiography images of a head and neck of the patient.

These and other advantages of the invention will be apparent to those of ordinary skill in the art by reference to the following detailed description and the accompanying drawings.

BRIEF DESCRIPTION OF THE DRAWINGS

Figure 1 shows a method for generating a patient-specific vascular tree, in accordance with one or more embodiments;

Figure 2 shows an example of a manually identified vascular tree, in accordance with one or more embodiments;

Figure 3 shows an example of a segmented manually identified vascular tree, in accordance with one or more embodiments;

Figure 4 shows aligned segmented manually identified vascular trees, in accordance with one or more embodiments;

Figure 5 shows segmented manually identified vascular trees aligned to mean vascular landmarks, in accordance with one or more embodiments;

Figure 6 shows a vascular tree template, in accordance with one or more embodiments;

Figure 7 shows an exemplary artificial neural network that may be used to implement one or more embodiments;

Figure 8 shows a convolutional neural network that may be used to implement one or more embodiments;

Figure 9 shows a schematic structure of a recurrent machine learning model that may be used to implement one or more embodiments; and

Figure 10 shows a high-level block diagram of a computer that may be used to implement one or more embodiments.

DETAILED DESCRIPTION

The present invention generally relates to methods and systems for deep reinforcement learning based robust neurovascular mapping. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. Further, reference herein to pixels of an image may refer equally to voxels of an image and vice versa.

Embodiments described herein provide for a novel AI/ML system that employs a deep reinforcement learning approach to extract the neurovascular tree from medical images. The system comprises two components: 1) the generation of a semantic vascular tree template with the anterior and posterior neurovascular systems, and 2) a deep reinforcement learning pipeline to adapt the semantic neurovascular tree template to medical imaging of a particular patient. Advantageously, embodiments described herein provide for the generation of a neurovascular mapping with enhanced accuracy and robustness, offering a better solution for the localization, visualization, and treatment planning of neurovascular abnormalities as compared to conventional approaches.

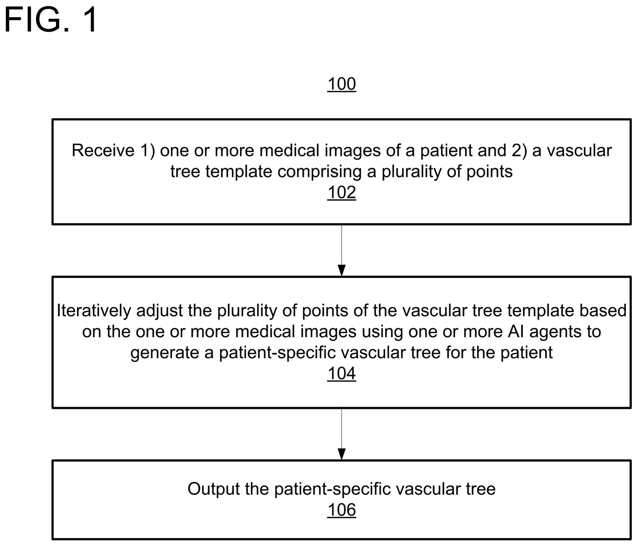

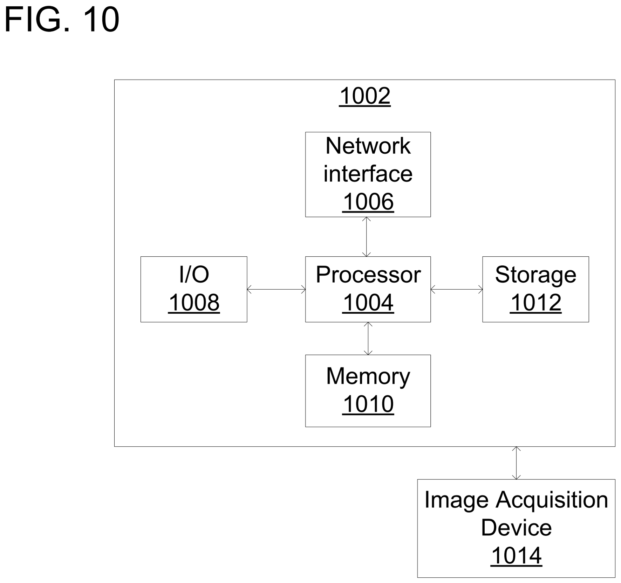

Figure 1 shows a method 100 for generating a patient-specific vascular tree, in accordance with one or more embodiments. The steps and sub-steps of method 100 may be performed by one or more suitable computing devices, such as, e.g., computer 1002 of Figure 10.

At step 102 of Figure 1, 1) one or more medical images of a patient and 2) a vascular tree template comprising a plurality of points are received. The one or more medical images depict vessels of the patient. For example, the one or more medical images may depict vessels in the head and neck regions of the patient. However, the one or more medical images may depict vessels in any other region of interest of the patient.

In one embodiment, the one or more medical images are CTA (computed tomography angiography) images of the patient. However, the one or more medical images may be of any other suitable modality, such as, e.g., MRI (magnetic resonance imaging), US (ultrasound), x-ray, or any other medical imaging modality or combinations of medical imaging modalities. The one or more medical images may be 2D (two dimensional) images and/or 3D (three dimensional) volumes, and may comprise a single medical image or a plurality of medical images. The one or more medical images may comprise image patches extracted from a larger medical image.

The vascular tree template represents a generic, non-patient-specific template of a vascular tree. The vascular tree template comprises vascular landmarks (e.g., aortic arch center, basilar artery branch, vertebral artery merge to basilar artery, bilateral common carotid artery, bilateral carotid bifurcation, bilateral carotid enter skull, bilateral carotid intracranial, bilateral carotid frontal, bilateral middle cerebral artery branch, bilateral middle cerebral artery M2 branch, bilateral middle cerebral artery M2 1/2 distal, bilateral carotid artery merge, bilateral anterior cerebral artery distal, bilateral posterior cerebral artery distal, and/or bilateral vertebral artery C1/C3/C5) and vascular vessel segments (e.g., bilateral carotid artery to aorta, bilateral vertebral artery to aorta, bilateral internal carotid artery, bilateral anterior cerebral artery A1/A2, bilateral middle cerebral artery M1/M2, basilar artery, bilateral vertebral artery C1/C3/C5, and/or bilateral posterior cerebral artery). The vascular tree template may be generated using any suitable approach.

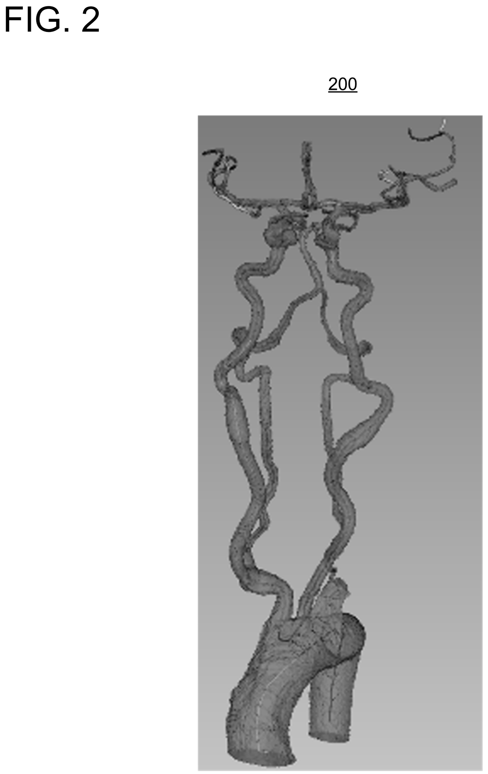

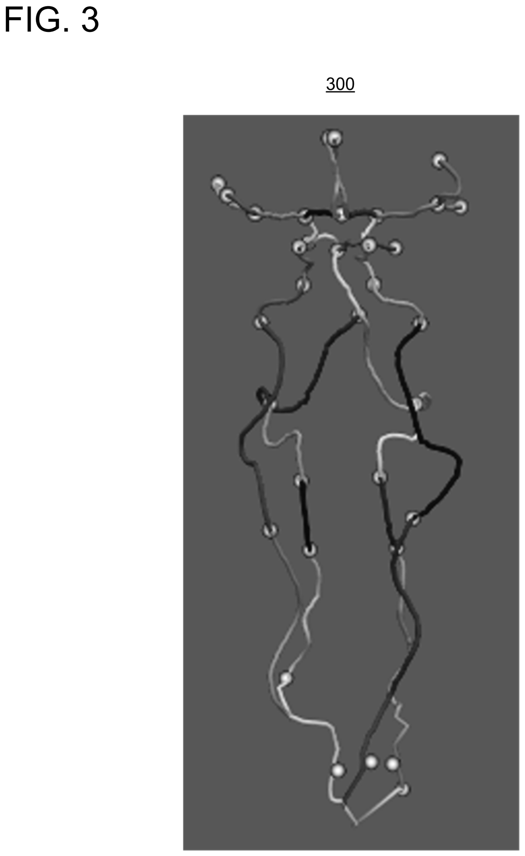

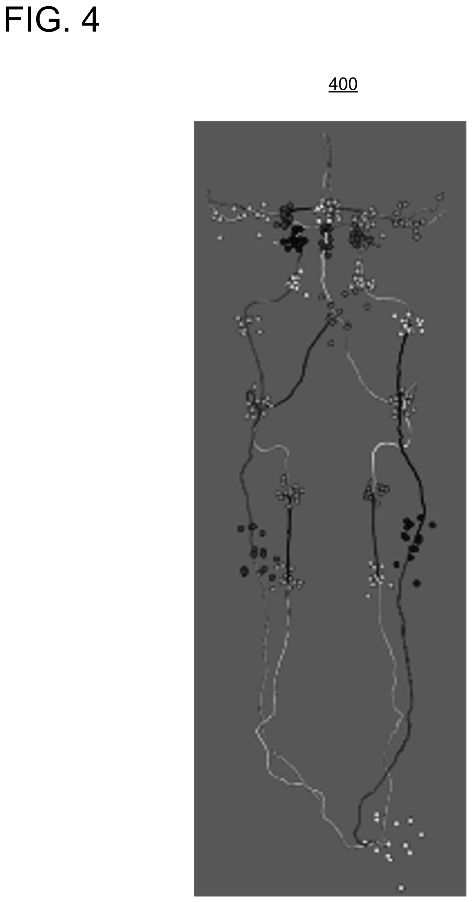

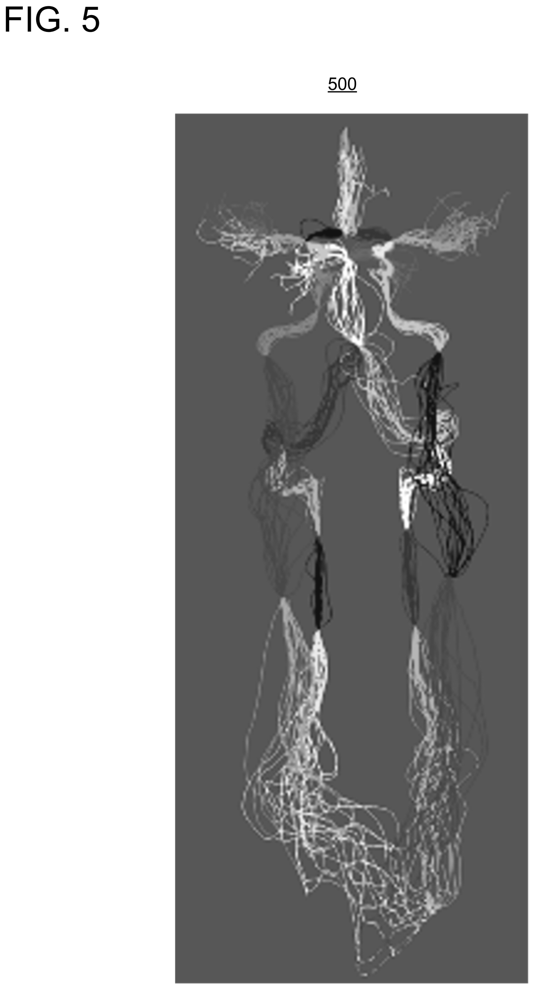

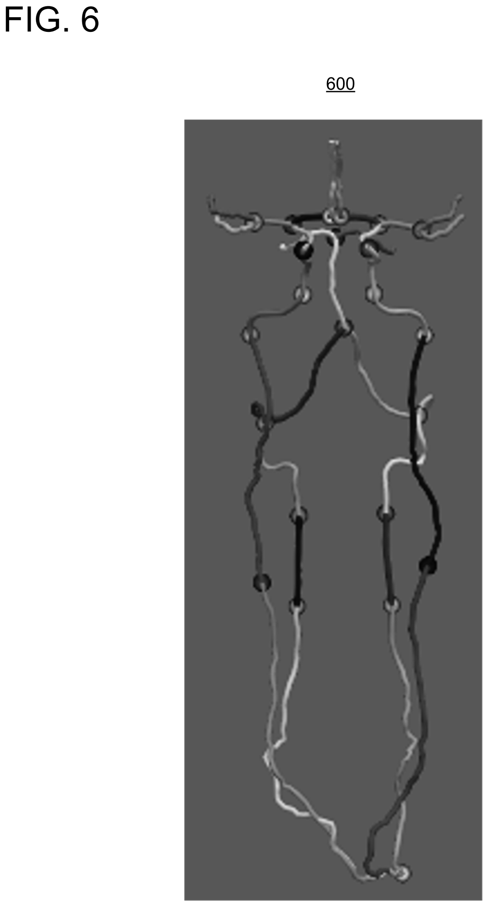

In one embodiment, the vascular tree template is generated by averaging a set of manually identified vascular trees from medical images for a patient population. In this embodiment, a set of vascular trees is manually identified from medical images of the patient population. Figure 2 shows an example of a manually identified vascular tree 200, in accordance with one or more embodiments. Vascular landmarks are then identified in each of the manually identified vascular trees, e.g., using any suitable (e.g., well-known) approach, and vessel segments of the manually identified vascular trees are identified by tracing the vessel centerline between corresponding landmarks. Figure 3 shows an example of a segmented manually identified vascular tree 300, in accordance with one or more embodiments. A template building process is then applied to the segmented manually identified vascular trees to generate the vascular tree template. First, one of the segmented manually identified vascular trees is randomly selected and the centerlines of the vessels of all other segmented manually identified vascular trees are aligned to the centerlines of the vessels of the selected segmented manually identified vascular tree, e.g., using rigid landmark matching. Figure 4 shows aligned segmented manually identified vascular trees 400, in accordance with one or more embodiments. Second, positions of each vascular landmark for the aligned segmented manually identified vascular trees are averaged to generate mean vascular landmarks. Third, all segmented manually identified vascular trees are aligned to the mean vascular landmarks, e.g., using any suitable (e.g., well-known) landmark matching approach. Figure 5 shows segmented manually identified vascular trees 500 aligned to mean vascular landmarks, in accordance with one or more embodiments. Finally, the centerlines of all vascular trees are averaged for each of the segments to generate the vascular tree template. Figure 6 shows a vascular tree template 600, in accordance with one or more embodiments.

The one or more medical images and/or the vascular tree template may be received, for example, by directly receiving the one or more medical images from an image acquisition device (e.g., image acquisition device 1014 of Figure 10) as the medical images are acquired, by loading the one or more medical images and/or the vascular tree template from a storage or memory of a computer system (e.g., storage 1012 or memory 1010 of computer 1002 of Figure 10), or by receiving the one or more medical images and/or the vascular tree template from a remote computer system (e.g., computer 1002 of Figure 10). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, the plurality of points of the vascular tree template is iteratively adjusted based on the one or more medical images using one or more AI agents to generate a patient-specific vascular tree for the patient.

In one embodiment, a preprocessing step is first performed on the one or more medical images to remove bone (e.g., using a deep image-to-image network) and automatically extract vascular landmarks (e.g., using any (e.g., well-known) landmark detection approach) from the one or more medical images. The vascular tree template is then aligned to the patient's space based on the preprocessed one or more medical images using piecewise landmark matching, providing for an initial vascular tree for the patient.

The AI agent is trained with deep reinforcement learning to jointly optimize each of the plurality of points of the initial vascular tree, instead of optimizing one landmark or point at a time. The AI agent computes optimal stepwise paths from each of the plurality of points on the initial vascular tree to predicted locations. In each path, the action at each step comprises movement of the N plurality of points on the initial vascular tree. The movement may comprise a movement in one of, for example, a left direction, a right direction, an inferior direction, a superior direction, a posterior direction, or an anterior direction. The choice of step direction or path termination is determined by the AI agent. The AI agent may be implemented according to any suitable neural network architecture. The AI agent receives as input at least one of the one or more medical images corresponding to a current position of the AI agent and generates as output a 7xN dimensional vector, where 7 corresponds to the six step directions or path termination of all of the plurality of points and N corresponds to the number of points in the plurality of points.

The AI agent is trained using deep reinforcement learning with temporal difference Q-learning using a DQN (deep Q-network) to find the path from the initial vascular tree to the target locations of the points, which are manually annotated in the training set. The AI agent predicts the agent Q-values (corresponding to the actions) and the DQN minimizes the distance between the target Q-values and the predicted Q-values as a function of network parameters according to a loss function (e.g., Q-learning loss). The L2 norm of the predicted movements of neighboring points can be added to the loss function to promote smoothness of the trajectory.

In one embodiment, to speed up convergence and avoid local minimum, a multi-scale strategy can be adopted to locate the path. The search starts at a coarse scale (e.g., 8 millimeters per voxel) and is progressively repeated on volumes with increasing resolution (e.g., two times the resolution, such as, 4 millimeters, 2 millimeters, 1 millimeters). The vascular tree is considered to be successfully fitted based on detection convergence for each scale.

In one embodiment, a plurality of AI agents may be applied for generating the patient-specific vascular tree to reduce memory consumption. For example, the plurality of AI agents may be applied where the image region of the vascular tree is too large to fit into memory of the computing device (e.g., graphical processing unit) on which the AI agents are executed. Each of the plurality of AI agents may be applied for generating different regions of the patient-specific vascular tree. In one example, the different regions may comprise the head region (e.g., superior to the carotid artery entering the skull) and the neck region (e.g., superior to the aorta arch and inferior to the carotid artery entering the skull) of the patient. This approach reduces the input size for each model to a fraction of the imaging volume, significantly decreasing the memory consumption during both training and inference.

In one embodiment, the AI agent may be trained for generating the patient-specific vascular tree to mitigate signals of vessels that are not of interest (e.g., external carotid artery and vein) in the one or more medical images. Such vessels that are not of interest may distract the deep reinforcement learning pipeline in adjusting the plurality of points of the vascular tree template. In one embodiment, the one or more medical images comprises one or more bone removed medical images (e.g., where the bone is removed from the one or more medical images using a machine learning based bone removal network) and the AI agent is trained to sample the environment from the one or more bone removed medical images . In another embodiment, the one or more medical images comprises one or more vessel probability maps (e.g., generated by a separate deep-learning segmentation model) and the AI agent is trained to sample the environment from the one or more vessel probability maps.

At step 106 of Figure 1, the patient-specific vascular tree is output. For example, the results of the one or more medical imaging analysis tasks can be output by displaying the results on a display device of a computer system (e.g., I/O 1008 of computer 1002 of Figure 10), storing the results on a memory or storage of a computer system (e.g., memory 1010 or storage 1012 of computer 1002 of Figure 10), or by transmitting the results to a remote computer system (e.g., computer 1002 of Figure 10).

In one embodiment, the one or more AI agents may be trained using synthesized pathological data, such as, e.g., for large vessel occlusions or aneurysms. By using synthesized pathological data, the one or more AI agents provide a more reliable estimation of the location of vessel segments. The synthesized pathological data is generated by simulating the pathological cases. The generation of synthesized large vessel occlusion data and synthesized aneurysm data are discussed below. Similar strategies may be applied for other types of pathologies.

To generate the synthesized large vessel occlusion data, an occlusion is randomly inserted into a particular segment of a vessel tree. The segment and centerline annotation of segments downstream of the segment in the corresponding segment is removed to simulate the cessation of blood flow. If the large vessel occlusion segment is the sole blood supply to the downstream segments of the vascular tree, the corresponding downstream segments are removed. For example, if a large vessel occlusion is inserted in the internal carotid artery, the downstream internal carotid segment is removed, but the bilateral middle cerebral artery M1 and M2 are retained as there are other sources of blood supply. However, if a large vessel occlusion is inserted in M1, the rest of M1 along with M2 is removed to simulate a realistic situation. The intensity of the corresponding removed regions is non-linearly transformed to match the intensity of the surrounding brain tissue.

Aneurysms typically appear as small surface lesion or restricted enlargements of the vascular tree. An aneurysm is inserted into the vessel segmentation as a small lesion on the surface of local enlargement. Both the size and location of the lesion are random. Although the segmentation is modified, the centerline annotation is unchanged to allow the AI agents to identify the true centerline in this pathological case. The intensity of the inserted area is set to the intensity of the attached vessel with a small amount of smoothing on the edge of the simulated lesion.

The synthesized pathological data is combined to augment the original training data to train the one or more AI agents. For missing neurovascular segments in the large vessel occlusion cases, smaller weights are assigned to corresponding portions in the vascular trees. This allows the AI agents to make a reasonable estimation of the missing vascular tree without confusing it by trying to overfit the prediction to synthesized pathological data. For aneurysms, more weights to the tree around the simulated lesions is assigned to guide the AI agents in correctly fitting the vascular tree.

Advantageously, embodiments described herein provide for completeness in the generated patient-specific vascular tree. Specifically, the generated patient-specific vascular tree can represent the entire tree even for vascular components that are not present due to natural anatomical differences or disease. This is important for visualizing the vascular tree, pinpointing disease, and planning medical interventions. Further, embodiments described herein provide robustness. The AI agents and the vessel segmentation network, trained using synthesized pathological data, have been found to be more resilient to disease situations. This leads to better detection of disease-related changes, improving the accuracy of diagnoses. In addition, embodiments described herein provide for efficiency. Embodiments described herein are based on deep neural networks, which have fast run time when deployed. The joint optimization of all points of the vascular tree also allows faster inference. This is important as some severe vascular conditions may require fast responses.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., the one or more AI agents utilized at step 104 of Figure 1, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

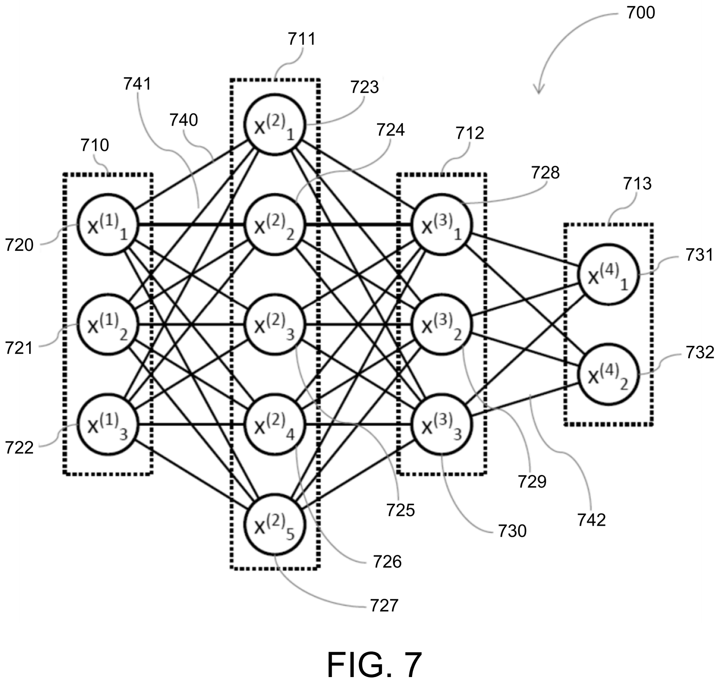

Figure 7 shows an embodiment of an artificial neural network 700 that may be used to implement one or more machine learning models described herein. Alternative terms for "artificial neural network" are "neural network", "artificial neural net" or "neural net".

The artificial neural network 700 comprises nodes 720, ..., 732 and edges 740, ..., 742, wherein each edge 740, ..., 742 is a directed connection from a first node 720, ..., 732 to a second node 720, ..., 732. In general, the first node 720, ..., 732 and the second node 720, ..., 732 are different nodes 720, ..., 732, it is also possible that the first node 720, ..., 732 and the second node 720, ..., 732 are identical. For example, in Figure 7 the edge 740 is a directed connection from the node 720 to the node 723, and the edge 742 is a directed connection from the node 730 to the node 732. An edge 740, ..., 742 from a first node 720, ..., 732 to a second node 720, ..., 732 is also denoted as "ingoing edge" for the second node 720, ..., 732 and as "outgoing edge" for the first node 720, ..., 732.

In this embodiment, the nodes 720, ..., 732 of the artificial neural network 700 can be arranged in layers 710, ..., 713, wherein the layers can comprise an intrinsic order introduced by the edges 740, ..., 742 between the nodes 720, ..., 732. In particular, edges 740, ..., 742 can exist only between neighboring layers of nodes. In the displayed embodiment, there is an input layer 710 comprising only nodes 720, ..., 722 without an incoming edge, an output layer 713 comprising only nodes 731, 732 without outgoing edges, and hidden layers 711, 712 in-between the input layer 710 and the output layer 713. In general, the number of hidden layers 711, 712 can be chosen arbitrarily. The number of nodes 720, ..., 722 within the input layer 710 usually relates to the number of input values of the neural network, and the number of nodes 731, 732 within the output layer 713 usually relates to the number of output values of the neural network.

In particular, a (real) number can be assigned as a value to every node 720, ..., 732 of the neural network 700. Here, $x^{(n)}_i$ denotes the value of the i-th node 720, ..., 732 of the n-th layer 710, ..., 713. The values of the nodes 720, ..., 722 of the input layer 710 are equivalent to the input values of the neural network 700, the values of the nodes 731, 732 of the output layer 713 are equivalent to the output value of the neural network 700. Furthermore, each edge 740, ..., 742 can comprise a weight being a real number, in particular, the weight is a real number within the interval [-1, 1] or within the interval [0, 1]. Here, $w^{(m,n)}_{i,j}$ denotes the weight of the edge between the i-th node 720, ..., 732 of the m-th layer 710, ..., 713 and the j-th node 720, ..., 732 of the n-th layer 710, ..., 713. Furthermore, the abbreviation $w^{(n)}_{i,j}$ is defined for the weight $w^{(n,n+1)}_{i,j}$.

In particular, to calculate the output values of the neural network 700, the input values are propagated through the neural network. In particular, the values of the nodes 720, ..., 732 of the (n+1)-th layer 710, ..., 713 can be calculated based on the values of the nodes 720, ..., 732 of the n-th layer 710, ..., 713 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 710 are given by the input of the neural network 700, wherein values of the first hid-den layer 711 can be calculated based on the values of the input layer 710 of the neural network, wherein values of the second hidden layer 712 can be calculated based in the values of the first hidden layer 711, etc.

In order to set the values $w^{(m,n)}_{i,j}$ for the edges, the neural network 700 has to be trained using training data. In particular, training data comprises training input data and training output data (denoted as $t_i$). For a training step, the neural network 700 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 700 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein y is a learning rate, and the numbers $\delta^{(n)}_j$ can be recursively calculated as

$$\delta^{(n)}_j = \left( \sum_k \delta^{(n+1)_k} \cdot w^{(n+1)_{j,k}} \right) \cdot f' \left( \sum_i x^{(n)_i} \cdot w^{(n)_{i,j}} \right)$$

based on $\delta^{(n+1)}_j$, if the (n+1)-th layer is not the output layer, and

$$\delta^{(n)}_j = \left( x^{(n+1)_j} - t^{(n+1)_j} \right) \cdot f' \left( x^{(n)_i} \cdot w^{(n)_{i,j}} \right)$$

if the (n+1)-th layer is the output layer 713, wherein f is the first derivative of the activation function, and $t^{(n+1)}_j$ is the comparison training value for the j-th node of the output layer 713.

A convolutional neural network is a neural network that uses a convolution operation instead of general matrix multiplication in at least one of its layers (so-called "convolutional layer"). In particular, a convolutional layer performs a dot product of one or more convolution kernels with the convolutional layer's input data / image, wherein the entries of the one or more convolution kernels are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

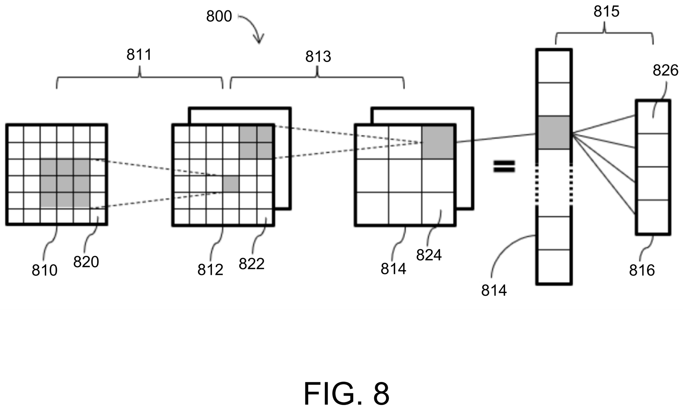

Figure 8 shows an embodiment of a convolutional neural network 800 that may be used to implement one or more machine learning models described herein. In the displayed embodiment, the convolutional neural network 800 comprises an input node layer 810, a convolutional layer 811, a pooling layer 813, a fully connected layer 814 and an output node layer 816, as well as hidden node layers 812, 814. Alternatively, the convolutional neural network 800 can comprise several convolutional layers 811, several pooling layers 813 and several fully connected layers 815, as well as other types of layers. The order of the layers can be chosen arbitrarily, usually fully connected layers 815 are used as the last layers before the output layer 816.

In particular, within a convolutional neural network 800 nodes 820, 822, 824 of a node layer 810, 812, 814 can be considered to be arranged as a d-dimensional matrix or as a d-dimensional image. In particular, in the two-dimensional case the value of the node 820, 822, 824 indexed with i and j in the n-th node layer 810, 812, 814 can be denoted as x(n)[i, j]. However, the arrangement of the nodes 820, 822, 824 of one node layer 810, 812, 814 does not have an effect on the calculations executed within the convolutional neural network 800 as such, since these are given solely by the structure and the weights of the edges.

A convolutional layer 811 is a connection layer between an anterior node layer 810 (with node values x(n-1)) and a posterior node layer 812 (with node values x(n)). In particular, a convolutional layer 811 is characterized by the structure and the weights of the incoming edges forming a convolution operation based on a certain number of kernels. In particular, the structure and the weights of the edges of the convolutional layer 811 are chosen such that the values x(n) of the nodes 822 of the posterior node layer 812 are calculated as a convolution x(n) = K * x(n-1) based on the values x(n-1) of the nodes 820 anterior node layer 810, where the convolution * is defined in the two-dimensional case as

$$x_k^{(n)}[i,j] = \left(K * x^{(n-1)}\right)[i,j] = \sum_{i'}\sum_{j'} K[i',j'] \cdot x^{(n-1)}[i\text{-}i', j\text{-}j'].$$

Here the kernel K is a d-dimensional matrix (in this embodiment, a two-dimensional matrix), which is usually small compared to the number of nodes 820, 822 (e.g., a 3x3 matrix, or a 5x5 matrix). In particular, this implies that the weights of the edges in the convolution layer 811 are not independent, but chosen such that they produce said convolution equation. In particular, for a kernel being a 3x3 matrix, there are only 9 independent weights (each entry of the kernel matrix corresponding to one independent weight), irrespectively of the number of nodes 820, 822 in the anterior node layer 810 and the posterior node layer 812.

In general, convolutional neural networks 800 use node layers 810, 812, 814 with a plurality of channels, in particular, due to the use of a plurality of kernels in convolutional layers 811. In those cases, the node layers can be considered as (d+1)-dimensional matrices (the first dimension indexing the channels). The action of a convolutional layer 811 is then a two-dimensional example defined as

$$x^{(n)_b}[i,j] = \sum_a K_{a,b} * x^{(n-1)a}[i,j] = \sum_a \sum_{i'}\sum_{j'} K_{a,b}[i',j'] \cdot x^{(n-1)a}[i\text{-}i',j\text{-}j']$$

where $x^{(n-1)}_a$ corresponds to the a-th channel of the anterior node layer 810, $x^{(n)}_b$ corresponds to the b-th channel of the posterior node layer 812 and $K_{a,b}$ corresponds to one of the kernels. If a convolutional layer 811 acts on an anterior node layer 810 with A channels and outputs a posterior node layer 812 with B channels, there are A·B independent d-dimensional kernels $K_{a,b}$.

In general, in convolutional neural networks 800 activation functions are used. In this embodiment ReLU (acronym for "Rectified Linear Units") is used, with R(z) = max(0, z), so that the action of the convolutional layer 811 in the two-dimensional example is

$$x^{(n)_b}[i,j] = R\left(\sum_a \left(K_{a,b} * x^{(n-1)a}\right)[i,j]\right)$$
$$= R\left(\sum_a \sum_{i'}\sum_{j'} K_{a,b}[i',j'] \cdot x^{(n-1)a}[i - i', j - j']\right)$$

It is also possible to use other activation functions, e.g., ELU (acronym for "Exponential Linear Unit"), LeakyReLU, Sigmoid, Tanh or Softmax.

In the displayed embodiment, the input layer 810 comprises 36 nodes 820, arranged as a two-dimensional 6x6 matrix. The first hidden node layer 812 comprises 72 nodes 822, arranged as two two-dimensional 6x6 matrices, each of the two matrices being the result of a convolution of the values of the input layer with a 3x3 kernel within the convolutional layer 811. Equivalently, the nodes 822 of the first hidden node layer 812 can be interpreted as arranged as a three-dimensional 2x6x6 matrix, wherein the first dimension correspond to the channel dimension.

The advantage of using convolutional layers 811 is that spatially local correlation of the input data can exploited by enforcing a local connectivity pattern between nodes of adjacent layers, in particular by each node being connected to only a small region of the nodes of the preceding layer.

A pooling layer 813 is a connection layer between an anterior node layer 812 (with node values x(n-1)) and a posterior node layer 814 (with node values x(n)). In particular, a pooling layer 813 can be characterized by the structure and the weights of the edges and the activation function forming a pooling operation based on a non-linear pooling function f. For example, in the two-dimensional case the values x(n) of the nodes 824 of the posterior node layer 814 can be calculated based on the values x(n-1) of the nodes 822 of the anterior node layer 812 as

$$x^{(n)_b}[i,j] = f(x^{(n-1)}[id_1, jd_2], ..., x^{(n-1)b}[(i + 1)d_1\text{-}1, (j + 1)d_2\text{-}1])$$

In other words, by using a pooling layer 813 the number of nodes 822, 824 can be reduced, by re-placing a number d1·d2 of neighboring nodes 822 in the anterior node layer 812 with a single node 822 in the posterior node layer 814 being calculated as a function of the values of said number of neighboring nodes. In particular, the pooling function f can be the max-function, the average or the L2-Norm. In particular, for a pooling layer 813 the weights of the incoming edges are fixed and are not modified by training.

The advantage of using a pooling layer 813 is that the number of nodes 822, 824 and the number of parameters is reduced. This leads to the amount of computation in the network being reduced and to a control of overfitting.

In the displayed embodiment, the pooling layer 813 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 800 are fully connected layers 815. A fully connected layer 815 is a connection layer between an anterior node layer 814 and a posterior node layer 816. A fully connected layer 813 can be characterized by the fact that a majority, in particular, all edges between nodes 814 of the anterior node layer 814 and the nodes 816 of the posterior node layer are present, and wherein the weight of each of these edges can be adjusted individually.

In this embodiment, the nodes 824 of the anterior node layer 814 of the fully connected layer 815 are displayed both as two-dimensional matrices, and additionally as non-related nodes (indicated as a line of nodes, wherein the number of nodes was reduced for a better presentability). This operation is also denoted as "flattening". In this embodiment, the number of nodes 826 in the posterior node layer 816 of the fully connected layer 815 smaller than the number of nodes 824 in the anterior node layer 814. Alternatively, the number of nodes 826 can be equal or larger.

Furthermore, in this embodiment the Softmax activation function is used within the fully connected layer 815. By applying the Softmax function, the sum the values of all nodes 826 of the output layer 816 is 1, and all values of all nodes 826 of the output layer 816 are real numbers between 0 and 1. In particular, if using the convolutional neural network 800 for categorizing input data, the values of the output layer 816 can be interpreted as the probability of the input data falling into one of the different categories.

In particular, convolutional neural networks 800 can be trained based on the backpropagation algorithm. For preventing overfitting, methods of regularization can be used, e.g., dropout of nodes 820, ..., 824, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

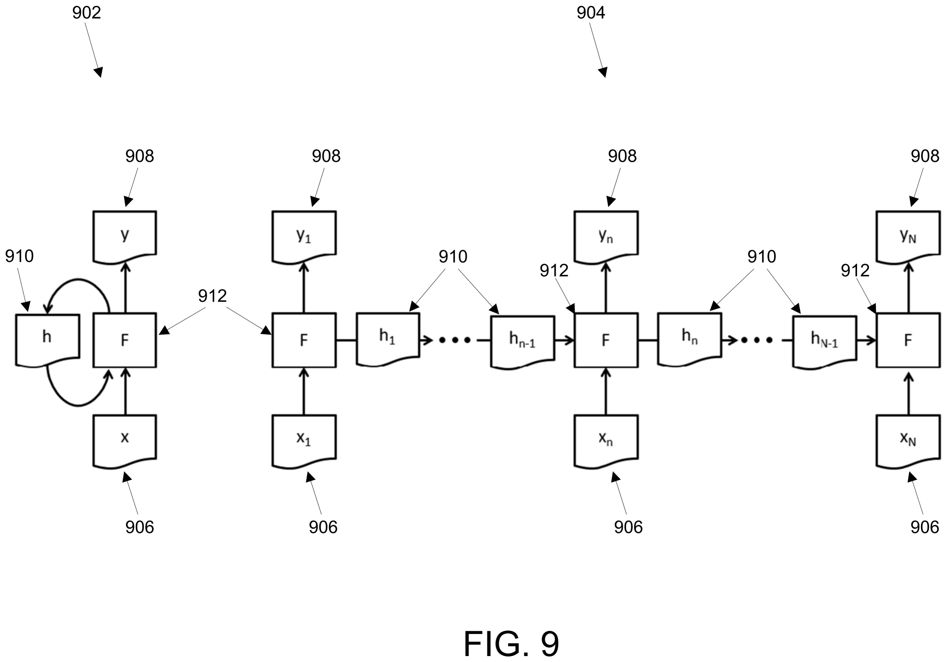

Figure 9 shows the schematic structure of a recurrent machine learning model F, both in a recurrent representation 902 and in an unfolded representation 904, that may be used to implement one or more machine learning models described herein. The recurrent machine learning model takes as input several input datasets x, $x_1$, ..., $x_N$ 906 and creates a corresponding set of output datasets y, $y_1$, ..., $y_N$ 908. Furthermore, the output depends on a so-called hidden vector h, $h_1$, ..., $h_N$ 910, which implicitly comprises information about input datasets previously used as input for the recurrent machine learning model F 912. By using these hidden vectors h, $h_1$, ..., $h_N$ 910, a sequentiality of the input datasets can be leveraged.

In a single step of the processing, the recurrent machine learning model F 912 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 912 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 912 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(x_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figure 1. Certain steps or functions of the methods and workflows described herein, including one or more of the steps or functions of Figure 1, may be performed by a server or by another processor in a network-based cloud-computing system. Certain steps or functions of the methods and workflows described herein, including one or more of the steps of Figure 1, may be performed by a client computer in a network-based cloud computing system. The steps or functions of the methods and workflows described herein, including one or more of the steps of Figure 1, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figure 1, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 1002 that may be used to implement systems, apparatuses, and methods described herein is depicted in Figure 10. Computer 1002 includes a processor 1004 operatively coupled to a data storage device 1012 and a memory 1010. Processor 1004 controls the overall operation of computer 1002 by executing computer program instructions that define such operations. The computer program instructions may be stored in data storage device 1012, or other computer readable medium, and loaded into memory 1010 when execution of the computer program instructions is desired. Thus, the method and workflow steps or functions of Figure 1 can be defined by the computer program instructions stored in memory 1010 and/or data storage device 1012 and controlled by processor 1004 executing the computer program instructions. For example, the computer program instructions can be implemented as computer executable code programmed by one skilled in the art to perform the method and workflow steps or functions of Figure 1. Accordingly, by executing the computer program instructions, the processor 1004 executes the method and workflow steps or functions of Figure 1. Computer 1002 may also include one or more network interfaces 1006 for communicating with other devices via a network. Computer 1002 may also include one or more input/output devices 1008 that enable user interaction with computer 1002 (e.g., display, keyboard, mouse, speakers, buttons, etc.).

Processor 1004 may include both general and special purpose microprocessors, and may be the sole processor or one of multiple processors of computer 1002. Processor 1004 may include one or more central processing units (CPUs), for example. Processor 1004, data storage device 1012, and/or memory 1010 may include, be supplemented by, or incorporated in, one or more application-specific integrated circuits (ASICs) and/or one or more field programmable gate arrays (FPGAs).

Data storage device 1012 and memory 1010 each include a tangible non-transitory computer readable storage medium. Data storage device 1012, and memory 1010, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 1008 may include peripherals, such as a printer, scanner, display screen, etc. For example, input/output devices 1008 may include a display device such as a cathode ray tube (CRT) or liquid crystal display (LCD) monitor for displaying information to the user, a keyboard, and a pointing device such as a mouse or a trackball by which the user can provide input to computer 1002.

An image acquisition device 1014 can be connected to the computer 1002 to input image data (e.g., medical images) to the computer 1002. It is possible to implement the image acquisition device 1014 and the computer 1002 as one device. It is also possible that the image acquisition device 1014 and the computer 1002 communicate wirelessly through a network. In a possible embodiment, the computer 1002 can be located remotely with respect to the image acquisition device 1014.

Any or all of the systems, apparatuses, and methods discussed herein may be implemented using one or more computers such as computer 1002.

One skilled in the art will recognize that an implementation of an actual computer or computer system may have other structures and may contain other components as well, and that Figure 10 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention as defined by the claims.

The following is a list of non-limiting illustrative embodiments disclosed herein:

Illustrative embodiment 1. A computer-implemented method comprising: receiving 1) one or more medical images of a patient and 2) a vascular tree template comprising a plurality of points; iteratively adjusting the plurality of points of the vascular tree template based on the one or more medical images using one or more AI (artificial intelligence) agents to generate a patient-specific vascular tree for the patient; and outputting the patient-specific vascular tree.

Illustrative embodiment 2. The computer-implemented method of illustrative embodiment 1, wherein iteratively adjusting the plurality of points of the vascular tree template based on the one or more medical images using one or more AI (artificial intelligence) agents to generate a patient-specific vascular tree for the patient comprises: jointly adjusting the plurality of points at each iteration.

Illustrative embodiment 3. The computer-implemented method of any one of illustrative embodiments 1-2, wherein iteratively adjusting the plurality of points of the vascular tree template based on the one or more medical images using one or more AI (artificial intelligence) agents to generate a patient-specific vascular tree for the patient comprises: iteratively adjusting the plurality of points in at least one of a left direction, a right direction, an inferior direction, a superior direction, a posterior direction, or an anterior direction.

Illustrative embodiment 4. The computer-implemented method of any one of illustrative embodiments 1-3, wherein the one or more AI agents receives as input at least one of the one or more medical images corresponding to a current position of the one or more AI agents and generates as output a vector corresponding to the adjustments for the plurality of points.

Illustrative embodiment 5. The computer-implemented method of any one of illustrative embodiments 1-4, wherein the one or more medical images comprise at least one of one or more bone removed medical images or one or more vessel probability maps.

Illustrative embodiment 6. The computer-implemented method of any one of illustrative embodiments 1-5, wherein the one or more AI agents are trained using synthesized large vessel occlusion data, the synthesized large vessel occlusion data generated by simulating an occlusion in a particular segment of a vessel tree by: removing the particular segment from the vessel tree; and in response to determining that the particular segment is a sole bloody supply to a downstream segment of the vascular tree, removing the downstream segment from the vascular tree.

Illustrative embodiment 7. The computer-implemented method of any one of illustrative embodiments 1-6, further comprising: generating the vascular tree template by averaging a set of manually identified vascular trees for a patient population.

Illustrative embodiment 8. The computer-implemented method of illustrative embodiment 7, wherein generating the vascular tree template by averaging a set of manually identified vascular trees for a patient population comprises: selecting a vascular tree from the set of manually identified vascular trees; aligning the remaining vascular trees of the set of manually identified vascular trees to the selected vascular tree; averaging positions of each vascular landmark of the aligned vascular trees; and aligning the set of manually identified vascular trees to the average position of each vascular landmark.

Illustrative embodiment 9. The computer-implemented method of any one of illustrative embodiments 1-8, wherein the one or more medical images are computed tomography angiography images of a head and neck of the patient.

Illustrative embodiment 10. An apparatus comprising: means for receiving 1) one or more medical images of a patient and 2) a vascular tree template comprising a plurality of points; means for iteratively adjusting the plurality of points of the vascular tree template based on the one or more medical images using one or more AI (artificial intelligence) agents to generate a patient-specific vascular tree for the patient; and means for outputting the patient-specific vascular tree.

Illustrative embodiment 11. The apparatus of illustrative embodiment 10, wherein the means for iteratively adjusting the plurality of points of the vascular tree template based on the one or more medical images using one or more AI (artificial intelligence) agents to generate a patient-specific vascular tree for the patient comprises: means for jointly adjusting the plurality of points at each iteration.

Illustrative embodiment 12. The apparatus of any one of illustrative embodiments 10-11, wherein the means for iteratively adjusting the plurality of points of the vascular tree template based on the one or more medical images using one or more AI (artificial intelligence) agents to generate a patient-specific vascular tree for the patient comprises: means for iteratively adjusting the plurality of points in at least one of a left direction, a right direction, an inferior direction, a superior direction, a posterior direction, or an anterior direction.

Illustrative embodiment 13. The apparatus of any one of illustrative embodiments 10-12, wherein the one or more AI agents receives as input at least one of the one or more medical images corresponding to a current position of the one or more AI agents and generates as output a vector corresponding to the adjustments for the plurality of points.

Illustrative embodiment 14. The apparatus of any one of illustrative embodiments 10-13, wherein the one or more medical images comprise at least one of one or more bone removed medical images or one or more vessel probability maps.

Illustrative embodiment 15. A non-transitory computer-readable storage medium comprising instructions which, when executed by a computer, cause the computer to carry out operations comprising: receiving 1) one or more medical images of a patient and 2) a vascular tree template comprising a plurality of points; iteratively adjusting the plurality of points of the vascular tree template based on the one or more medical images using one or more AI (artificial intelligence) agents to generate a patient-specific vascular tree for the patient; and outputting the patient-specific vascular tree.

Illustrative embodiment 16. The non-transitory computer-readable storage medium of illustrative embodiment 15, wherein iteratively adjusting the plurality of points of the vascular tree template based on the one or more medical images using one or more AI (artificial intelligence) agents to generate a patient-specific vascular tree for the patient comprises: jointly adjusting the plurality of points at each iteration.

Illustrative embodiment 17. The non-transitory computer-readable storage medium of any one of illustrative embodiments 15-16, wherein the one or more AI agents are trained using synthesized large vessel occlusion data, the synthesized large vessel occlusion data generated by simulating an occlusion in a particular segment of a vessel tree by: removing the particular segment from the vessel tree; and in response to determining that the particular segment is a sole bloody supply to a downstream segment of the vascular tree, removing the downstream segment from the vascular tree.

Illustrative embodiment 18. The non-transitory computer-readable storage medium of any one of illustrative embodiments 15-17, the operations further comprising: generating the vascular tree template by averaging a set of manually identified vascular trees for a patient population.

Illustrative embodiment 19. The non-transitory computer-readable storage medium of illustrative embodiment 18, wherein generating the vascular tree template by averaging a set of manually identified vascular trees for a patient population comprises: selecting a vascular tree from the set of manually identified vascular trees; aligning the remaining vascular trees of the set of manually identified vascular trees to the selected vascular tree; averaging positions of each vascular landmark of the aligned vascular trees; and aligning the set of manually identified vascular trees to the average position of each vascular landmark.

Illustrative embodiment 20. The non-transitory computer-readable storage medium of any one of illustrative embodiments 15-19, wherein the one or more medical images are computed tomography angiography images of a head and neck of the patient.

**Claims**

1. A computer-implemented method comprising:

   receiving (102) 1) one or more medical images of a patient and 2) a vascular tree template (600) comprising a plurality of points;
   iteratively adjusting (104) the plurality of points of the vascular tree template based on the one or more medical images using one or more AI (artificial intelligence) agents to generate a patient-specific vascular tree for the patient; and
   outputting (106) the patient-specific vascular tree.

2. The computer-implemented method of claim 1, wherein iteratively adjusting the plurality of points of the vascular tree template based on the one or more medical images using one or more AI (artificial intelligence) agents to generate a patient-specific vascular tree for the patient comprises:
   jointly adjusting the plurality of points at each iteration.

3. The computer-implemented method of claim 1 or 2, wherein iteratively adjusting the plurality of points of the vascular tree template based on the one or more medical images using one or more AI (artificial intelligence) agents to generate a patient-specific vascular tree for the patient comprises:
   iteratively adjusting the plurality of points in at least one of a left direction, a right direction, an inferior direction, a superior direction, a posterior direction, or an anterior direction.

4. The computer-implemented method of any one of claims 1 - 3, wherein the one or more AI agents receives as input at least one of the one or more medical images corresponding to a current position of the one or more AI agents and generates as output a vector corresponding to the adjustments for the plurality of points.

5. The computer-implemented method of any one of claims 1 - 4, wherein the one or more medical images comprise at least one of one or more bone removed medical images or one or more vessel probability maps.

6. The computer-implemented method of any one of claims 1 - 5, wherein the one or more AI agents are trained using synthesized large vessel occlusion data, the synthesized large vessel occlusion data generated by simulating an occlusion in a particular segment of a vessel tree by:

   removing the particular segment from the vessel tree; and
   in response to determining that the particular segment is a sole bloody supply to a downstream segment of the vascular tree, removing the downstream segment from the vascular tree.

7. The computer-implemented method of any one of claims 1 - 6, further comprising:
   generating the vascular tree template by averaging a set of manually identified vascular trees for a patient population.

8. The computer-implemented method of claim 7, wherein generating the vascular tree template by averaging a set of manually identified vascular trees for a patient population comprises:

   selecting a vascular tree from the set of manually identified vascular trees (200);
   aligning (400) the remaining vascular trees of the set of manually identified vascular trees to the selected vascular tree;

averaging positions of each vascular landmark of the aligned vascular trees; and

aligning (500) the set of manually identified vascular trees to the average position of each vascular landmark.

9. The computer-implemented method of any one of claims 1 - 8, wherein the one or more medical images are computed tomography angiography images of a head and neck of the patient.

10. An apparatus comprising:

means for receiving (102) 1) one or more medical images of a patient and 2) a vascular tree template (600) comprising a plurality of points;

means for iteratively adjusting (104) the plurality of points of the vascular tree template based on the one or more medical images using one or more AI (artificial intelligence) agents to generate a patient-specific vascular tree for the patient; and

means for outputting (106) the patient-specific vascular tree.

11. The apparatus of claim 10, wherein the means for iteratively adjusting the plurality of points of the vascular tree template based on the one or more medical images using one or more AI (artificial intelligence) agents to generate a patient-specific vascular tree for the patient comprises:

means for jointly adjusting the plurality of points at each iteration; and/or

means for iteratively adjusting the plurality of points in at least one of a left direction, a right direction, an inferior direction, a superior direction, a posterior direction, or an anterior direction.

12. The apparatus of claim 10 or 11, wherein the one or more AI agents receives as input at least one of the one or more medical images corresponding to a current position of the one or more AI agents and generates as output a vector corresponding to the adjustments for the plurality of points.

13. The apparatus of any one of claims 10 - 12, wherein

the one or more medical images comprise at least one of one or more bone removed medical images or one or more vessel probability maps; and/or

the one or more medical images are computed tomography angiography images of a head and neck of the patient.

14. The apparatus of any one of claims 10 - 13, further comprising means to perform the specific method steps of any one of claims 6 - 8.

15. A non-transitory computer-readable storage medium comprising instructions which, when executed by a computer, cause the computer to carry out the method of any one of claims 1 - 9.

# FIG. 1

100

```
Receive 1) one or more medical images of a patient and 2) a vascular
tree template comprising a plurality of points
102
```

```
Iteratively adjust the plurality of points of the vascular tree template based
on the one or more medical images using one or more AI agents to
generate a patient-specific vascular tree for the patient
104
```

```
Output the patient-specific vascular tree
106
```

# FIG. 2

200

# FIG. 3

300

# FIG. 4

400

# FIG. 5

500

# FIG. 6

600

FIG. 7

FIG. 8

FIG. 9

# FIG. 10

Europäisches
Patentamt

European
Patent Office

Office européen
des brevets

## EUROPEAN SEARCH REPORT

**Application Number**

EP 25 20 8227

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAN DE GIESSEN MARTIJN ET AL: "Probabilistic atlas based labeling of the cerebral vessel tree", PROGRESS IN BIOMEDICAL OPTICS AND IMAGING, SPIE - INTERNATIONAL SOCIETY FOR OPTICAL ENGINEERING, BELLINGHAM, WA, US, vol. 9413, 20 March 2015 (2015-03-20), pages 94130V-94130V, XP060051123, ISSN: 1605-7422, DOI: 10.1117/12.2081604 ISBN: 978-1-5106-0027-0 | 1-5, 9-13,15 | INV. G16H30/40 A61B6/50 G06F18/214 G06N3/08 G06T7/00 G06V10/70 G06V40/14 G16H50/20 G16H50/50 G16H50/70 |
| Y | * The whole document, in particular: Pages 1, 2, 4, 5; Figures 1 - 3. * ----- | 6-8,14 | |
| X | EP 4 160 529 A1 (SIEMENS HEALTHCARE GMBH [DE]) 5 April 2023 (2023-04-05) | 1-5, 9-13,15 | |
| Y | * The whole document, in particular: Paragraphs [0004] - [0008], [0027], [0031], [0032], [0041], [0042], [0069]; Figures 1 - 4, 8, 10, 13. * ----- | 6-8,14 | |

**TECHNICAL FIELDS SEARCHED (IPC)**

G16H

The present search report has been drawn up for all claims

| Place of search | Date of completion of the search | Examiner |
|---|---|---|
| Munich | 17 February 2026 | Ruschke, Stefan |

EPO FORM 1503 03.82 (P04C01)

## ANNEX TO THE EUROPEAN SEARCH REPORT
## ON EUROPEAN PATENT APPLICATION NO.

EP 25 20 8227

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2026

| Patent document cited in search report | | Publication date | Patent family member(s) | | Publication date |
|---|---|---|---|---|---|
| EP 4160529 | A1 | 05-04-2023 | CN | 115880219 A | 31-03-2023 |
| | | | EP | 4160529 A1 | 05-04-2023 |
| | | | US | 2023102246 A1 | 30-03-2023 |

EPO FORM P0459

For more details about this annex : see Official Journal of the European Patent Office, No. 12/82